# EUROPEAN PATENT APPLICATION

(11) **EP 1 297 826 A1**
(43) Date of publication of application: **02.04.2003**
(21) Application number: 01123120.6
(22) Date of filing: 27.09.2001
(51) Int. Cl.: A61K 9/16, A61K 47/32

(54) **Nasal pharmaceutical compositions containing a NK-2 antagonist**

(71) Applicant: Menarini Ricerche S.p.A., 00040 Pomezia (IT); BERLIN-CHEMIE AG, 12489 Berlin (DE)
(72) Inventor: Criscuoli, Marco Menarini ricerche S.p.A., 00040 Pomezia, Roma (IT); Manzini, Stefano Menarini ricerche S.p.A., 00040 Pomezia, Roma (IT); Maggi, Carlo Alberto Menarini ricerche S.p.A., 00040 Pomezia, Roma (IT)
(74) Representative: Gervasi, Gemma, Dr.

(57) **Abstract**

Pharmaceutical compositions suitable for the nasal administration containing:
iii) an NK-2 antagonist or its pharmaceutically acceptable salts
iv) a polyacrylic acid and/or its derivatives
possibly in combination with other excipients used in the preparation of pharmaceutical composition for nasal administration are described.

## Description

### State of the art

The NK2 receptor of tachykinins is widely expressed in the peripheral nervous system of mammals. Among the various effects produced by selective stimulation of the NK-2 receptor there are modulation of smooth muscle contraction and of visceral pain sensitivity. NK-2 antagonists have been considered agents capable of controlling excessive contraction of the smooth muscle in any pathological condition, such as irritation or spasms, in which release of tachykinins concurs in the genesis of the disorder. In particular, the bronchospastic component of asthma, coughing, pulmonary irritations, intestinal irritation, intestinal spasms, local spasms of the bladder and the ureter during cystitis, renal infections and colics may be considered conditions in which the administration of NK-2 antagonists may be effective (A.L. Magnan et al, Neuropeptides, 1993, 24, 199). Recently, also the Irritable Bowel Syndrome (IBS) was considered as a possible therapeutic target for tachykinin antagonists. (M Camilleri, Gastroenterology, 2001, 120, 652). IBS is clinically characterized by chronic abdominal pain associated to disturbed bowel habits (constipation or/and diarrhea). Visceral sensory thresholds are generally lowered.

In the past the therapeutic approaches to resolve pain in IBS patients focused on restoration of the abnormal gut motility patterns with the use of antispasmodic agents, and either mass laxatives and prokinetic compounds or antidiarrheal agents. In recents years, visceral hyperalgesia has been recognised as the main pathophysiological event underlying IBS symptoms (EA Mayer & FG Gebhart, Gastroenterology, 1994, 107, 271). It is predicted that drugs able to correct visceral hyperalgesia will constitute an important step forward in IBS treatment.

NK-2 antagonists have been shown in animal models to be able to reduce visceral hypersensitivity induced by different stimuli (PM Anton et al, Pain, 2001, 92, 219; PG McLean et al, Eur J Pharmacol, 1997, 337, 279)

Several different compounds with NK-2 antagonist properties have been reported during the years in the patent literature. In WO 93/21227 NK-2 antagonists with a bicyclic scaffold were described; their extremely low water solubility hampered any possible pharmacological applications of such molecules up to now, notwithstanding their in vitro interesting biological activity.

In EP815126 NK-2 antagonists with the same bicyclic scaffold as in WO93/21227 were described, but these compounds had an hydrophilic moiety that made them more suitable for a pharmaceutical development. One of the molecules described in EP815126, nepadutant, corrects visceral hyperalgesia in animal models of IBS (M Toulouse, Br J Pharmacol, 2000, 129, 193).

Until now diseases characterized by irritation of the smooth musculature and pain have been generally treated with the following pharmaceutical forms:
- oral (tablets or suspensions, both as prompt and controlled release forms)
- parenteral (i.v., i.m., s.c.)

These administration routes may be not always applicable, due to some drawbacks:
- oral administration may suffer of : absorption problems, first-pass-effect, metabolization in the digestive system, slow response
- parenteral administration may suffer of: low compliance, difficulties in self-administration, local irritation.

All these problems may even increase due to certain characteristics of the products to be administered. In case of the NK-2 antagonists described in EP815126, their relatively low water solubility and their still poor oral bioavailability made both administration routes unsatisfactory. In particular, nepadutant showed a very poor oral bioavailability (1%) when administered both as a solution to rats (A Lippi et al, Drug Metab Disp, 1998, 26, 1077) and as dry powder in capsules to human volunteers.

Therefore there is a need to develop new pharmaceutical compositions with NK-2 antagonists in general and compounds of EP815126 in particular, in order to avoid the drawbacks of the aforementioned pharmaceutical forms and to compensate for the negative characteristics of the products.

### Detailed description of the invention

The purpose of the present invention is therefore to make available a new pharmaceutical composition for NK-2 antagonists in general and for the compounds described in EP815126 in particular. The composition disclosed in the present invention is a new nasal composition characterised by the presence of a polyacrylic acid derivative as an excipient.

Nasal route is a very interesting administration route, since it could avoid the drawbacks of the oral route, but it has not usually been chosen for the administration of NK-2 antagonists especially when these compounds have been used for the treatment of the pathologies of the gastrointestinal tract , and for IBS in particular. Indeed, preliminary data had shown that a good absoption (> 80%) could be obtained after instilling a concentrated nepadutant solution in the nose of rats (A Lippi et al, Drug Metab Disp, 1998, 26, 1077). Further experiments in rats, dogs and humans did not allow to reproduce these data neither with simple solutions in buffered saline, nor with formulated solutions containing solubility and viscosity enhancers. Maximum bioavailability obtained in these trials ranged from 20% in rats to 2% in dogs and humans (comparable results were obtained in these species, indicating that dogs can be a suitable model for nasal biavailability studies). Therefore, intranasally sprayable solutions were considered not to be a suitable way to solve bioavailability problems of nepadutant and related compounds.

The polyacrylic acid or its derivatives are well known materials used in many pharmaceutical applications (Dittgen, M et al, S.T.P. Pharma Sci. , 1997, 7 (6), 403-437). Their bio-adhesive characteristics were used also for the preparation of nasal pharmaceutical compositions:
- GB2042888, for formulations with slow release properties
- EP516141, for a gradual release of the active compound
- EP497956, for a controlled release formulation
- US4250163 and US4226848 for treatment of recurrent stomatic aphthosis.

Using the formulation of the present invention, composed by:
a) the active ingredient selected among the compounds described in EP815126
b) the polyacrylic acid and/or its derivatives
c) eventual inert excipients,
surprisingly, as an additional quality, a penetration supporting effect was observed. In fact, in animal experiments, an absolute bioavailability of up to 52% of the dose was found, while in similar experiments an oral or other intranasal formulations of different composition were bioavailable for a 3% maximum.

In EP815126 (pag 5, line 20), the possibility of a nasal composition is cited, but it is clear from the context that the nasal preparation is just an example of the possible pharmaceutical compositions that may be done with the compounds of the invention: there is no hint in the text that a nasal composition could have such a surprising effect on the total bioavailability of an active NK-2 antagonist.

This surprising increase in bioavailability of NK-2 antagonists is definitively due to the use of the polyacrylic acid and/or its derivatives in the nasal pharmaceutical compositions described in the present invention.

The object of the present invention is therefore a new pharmaceutical composition suitable for nasal administration containing:
a) an NK-2 antagonist
b) a polyacrylic acid and/or its derivatives
possibly in combination with the excipients normally used for the preparation of pharmaceutical compositions for nasal administration.

The NK-2 antagonist is selected among those described in EP815126, in particular byciclic compounds of general formula (I) 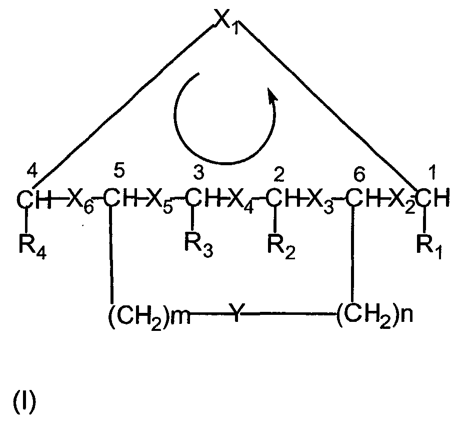 wherein:
X1, X2 , X3, X4, X5, and X6, same or different from one another, represent a - NR'CO- or a -CONR'- group, wherein R' is H or C1-3 alkyl;
Y represents a group selected from -NRCO-, -CONR-, or -SS- wherein R is H or C1-3 alkyl;
at least one of the R1, R2, R3 and R4 groups, same or different from one another, is hydrophilic and the remaining groups are hydrophobic; m and n, same or different from one another, are each an integer number from 1 to 4;

More particularly the hydrophobic groups can be separately selected from the following:
a) groups CnH2n+1 wherein n= 0, 1-4
b) linear- or branched alkyl groups corresponding to CnH2n-U-W wherein n= 1-4; U= 0, COO, CONH, S and W= C1-5 alkyl-, aryl or C1-5 alkyl-aryl-group containing from 1 to 15 carbon atoms and where aryl- is selected between phenyl or naphtyl
c) (CH2)n -C6H3-A-B wherein n= 0, 1-3; A and B, placed in any of the ortho, meta or para positions, same or different from one another, represent H, halogen, OR, NHR, NR2, CH3, SR wherein R is an C1-4 alkyl-, phenyl- or C1-4 alkyl-phenyl-group,
d) (CH2)n -C6H10 R', wherein n= 0, 1-3 and R'= H, C1-3 alkyl
e) (CH2)n -heterocycle, wherein n= 0, 1-3 and for heterocycle it is meant: imidazolyl-2-yl, indolyl-3-yl, furanyl-3-yl, pyridyl-3-yl, imidazolyl-3-yl
f) a -(CH2)s- group, wherein s= 3, 4, possibly OH-substituted or condensed with an aromatic group, which cyclizes with one of the two adjacent X1-6 groups in order to produce the side chain of proline, hydroxyproline, octahydroindol-2-carboxylic acid, tetrahydroisoquinolinic acid
g) the side chain of a natural hydrophobic amino acid selected from the group consisting of: glycine, alanine, valine, leucine, isoleucine, methionine, phenylalanine, tyrosine, tryptophan, proline, histidine, aspargine, glutamine.
h) the side chain of a natural hydrophilic amino acid, selected from the group consisting of: serine, threonine, cysteine, aspartic acid, glutamic acid, t-carboxyglutamic acid, arginine, ornithine, lysine, suitably substituted in order to render it hydrophobic through the formation of an esteric or an amidic bond with a C1-4 acyl group or a C1-3 alkyl group.
i) the side chain of non-natural hydrophobic amino acids selected from the group consisting of: norleucine, norvaline, alloisoleucine, cyclohexylglycine (Chg), α-amino-n-butyric acid (Aba), cyclohexylalanine (Cha), aminophenylbutyric acid (Pba), phenylalanines mono- and disubstituted in the ortho, meta and para positions of the benzene ring with one or more of the following groups: C1-10 alkyl, C1-10 alkoxy, halogen, β-2-thienylalanine, β-3-thienylalanine, β-2- furanyl alanine, β-3-furanylalanine, β-2-piridylalanine, β-3-pindylalanine, β-4-pindylalanine, β-(1-naphtyl)alanine, β-(2-naphtyl)alanine, O-C1-5alkylated serine- threonine- tyrosine-derivatives, S-C1-5 alkyl cysteine, S-C1-5alkyl homocysteine, N-C1-5 alkyl lysine, N-C1-5 alkyl ornithine, N-C1-5 alkyl 2,3 diaminopropionic acid.

Preferably the hydrophilic groups are chosen in the group L-Q wherein L is a chemical bond or a linear or branched C1-6 alkyl-group and Q is chosen in the group consisting of:
i) hydroxyl, amine, guanidine, carboxyl, sulfate, phosphonate, phosphate;
ii) linear, branched or cyclic C1-6 alkyl chain containing one or more hydroxyl, amine, guanidine, carboxyl, sulfate, phosphate;
iii) an aromatic group, selected between phenyl or naphtyl, mono-, di- or trisubstituted in the ortho-, meta-, para-position with hydroxyl, amino, guanidine, carboxyl, sulfate, phosphate;
iv) a group M, OM, CONHM, NHCOM wherein M is an hydrophilic group

Wherein the group M is chosen in the group consisting of:
a) possibly substituted mono-, di-, tri-glycosidic residues;
b) linear, branched or cyclic C1-6 alkyl-chains, containing one or more groups hydroxyl, amine, guanidine, carboxyl, sulfate, phosphonate, phosphate.

Particularly preferred are compounds wherein the glycosidic residues are selected from the group consisting of: D or L ribose, D or L arabinose, D or L xylose, D or L lyxose, D or L allose, D or L altrose, D or L glucose, D or L mannose, D or L gulose, D or L idose, D or L galactose,D or L talose, D or L allulose, D or L fructose, D or L sorbose, D or L tagatose; 5-deoxy-D or L-arabinose, 2-deoxy-D or L-glucose, 2-deoxy-D or L-galactose, 2-deoxy-D or L-arabinose, 2-deoxy-D or L-ribose, D or L fucose, D or L ramnose; D-glucosamine, D-mannosamine, D-galactosamine, daunosamine, acosamine and N-acylate derivative s thereof with lower fat acids, i.e. containing a N-formylic, acetylic, propionilic, butyric residue; glucuronic acid, galacturonic acid; cellobiose, lactose, maltose, D-lactosamine, cellotriose, maltotriose; tris(hydroxymethyl)methyl, D or L arabitol, D or L erythrol, D or L-perseitol, D or L ribitol, D or L sorbitol, D or L xylitol; or those where the group M is selected from the residue of tartaric acid, glucaric acid, gluconic acid, bycine, quinic acid, mucic acid, glucosaminic acid.

Preferred compounds of formula (I) as above defined are those wherein:
the hydrophobic group is the side chain of a natural amino acid chosen in the group consisting of: glycine, alanine, valine, leucine, isoleucine,
methionine, phenylalanine, tyrosine, tryptophan, proline, histidine,
aspargine, glutamine or the side chain of non-natural hydrophobic amino acids selected from the group consisting of: norleucine, norvaline, alloisoleucine, cyclohexylglycine (Chg), α-amino-n-butyric acid (Aba), cyclohexylalanine (Cha), aminophenylbutyric acid (Pba), phenylalanines mono- and di- substituted in the ortho, meta and para positions of the benzene ring with one or more of the following groups: C1-10 alkyl, C1-10 alkoxy, halogen; β-2-thienylalanine, β-3-thienylalanine, β-2- furanyl alanine, β-3-furanylalanine, β-2-piridylalanine, β-3-piridylalanine, β-4-piridylalanine, β-(1-naphtyl)alanine, β-(2-naphtyl)alanine,
and the hydrophilic group is a group L-Q wherein L is a chemical bond or a linear or branched C1-6 alkyl-group and Q is chosen in the group consisting of: M, OM, CONHM, NHCOM wherein M is chosen in the group consisting of:
i) quinyl, gluconyl, glucoryl, sorbitolyl, sulphobenzoyl, sulphophenyl, sulphopropionyl, arginyl, lysyl,
ii) possibly substituted mono-, di-, tri-glycosidic residues selected from the group consisting of: D or L ribose, D or L arabinose, D or L xylose, D or L lyxose, D or L allose, D or L altrose, D or L glucose, D or L mannose, D or L gulose, D or L idose, D or L galactose, D or L talose, D or L allulose, D or L fructose, D or L sorbose, D or L tagatose; 5-deoxy-D or L-arabinose, 2-deoxy-D or L-glucose, 2-deoxy-D or L-galactose, 2-deoxy-D or L-arabinose, 2-deoxy-D or L-ribose, D or L fucose, D or L ramnose; D-glucosamine, D-mannosamine, D-galactosamine, daunosamine, acosamine and N-acylate derivative s thereof with lower fat acids, i.e. containing a N-formylic, acetylic, propionilic, butyric residue; glucuronic acid, galacturonic acid; cellobiose, lactose, maltose, D-lactosamine, cellotriose, maltotriose;

Among the products of formula (I) as above indicated, the products wherein if one or both R1 and R4 groups are hydrophilic, both R2 and R3 are hydrophobic and viceversa, are preferred.

The following compounds of general formula (I) are specifically preferred;
1) cyclo([Asnβ-D-Glc)-Asp-Trp-Phe-Dap-Leu]cyclo(2β--5β-)) (EP0815126 example No. 1)
2) cyclo ([Asn (β--D-2-deoxy-2-amino-Glc)-Asp-Trp-Phe-Dap-Leu] cyclo (2β--5β-)) (EP0815126 example No. 3)
3) cyclo ( [Asn(β--D-2-deoxy-2-acetamido-Glc)-Asp-Trp-Phe-Dap-Leu]cyclo(2β--5β-)) (EP0815126 example No. 4)
4) cyclo([Asn(β--D-mannopyranosyl)-Asp-Trp-Phe-Dap-Leu] cyclo(2β--5β-)) (EP0815126 example No. 10)
5) cyclo([Asn(β--D-galactopyranosyl)-Asp-Trp-Phe-Dap-Leu] cyclo(2β--5β-)) (EP0815126 example No. 12)
6) cyclo ([Asn(1-deoxy-sorbitol-1-yl)-Asp-Trp-Phe-Dap-Leu] cyclo(2β--5β-)) (EP0815126 example No. 16)
7) cyclo ([Asn[(4-O-(a-D-Glc)-β-D-Glc)]-Asp-Trp-Phe-Dap- Leu] cyclo(2β--5β-)) (EP0815126 example No. 17)
8) cyclo ([Asn(D-2-deoxy-glucopyranos-2-yl)-Asp-Trp-Phe-Dap-Leu] cyclo(2β--5β-)) (EP0815126 example No. 20)
9) cyclo ([Asn(β--D-2-deoxy-mannopyranos-2-yl)-Asp-Trp-Phe-Dap- Leu] cyclo(2β--5β-)) (EP0815126 example No. 28)
10) cyclo ([Dap(Lysyl)-Asp-Trp-Phe-Dap-Leu] cyclo(2β--5β-)) (EP0815126 example No. 32)

Compound cyclo ( [Asn(β--D-2-deoxy-2-acetamido-Glc)-Asp-Trp-Phe-Dap-Leu]cyclo(2β--5β-)) (EP0815126 example No. 4) is the most preferred one; this compound is also known with its INN name 'Nepadutant'.

The formula (I) is given in order to simplify the understanding of the compounds described hereinafter and in the Examples of EP0815126 with their chemical name in particular in so far as the groups X1-6 and Y are concerned.

The groups X1-6 and Y are in fact defined according to the aminoacid- sequence from the formal N- to the C-terminus of the peptide as they are represented in the linear structure, therefore reading Formula no problem arises in the understanding of the linear structure as reported in the Examples.

A polyacrylic acid derivative according to the invention is selected among the compounds summarised in the monograph "Carbomers" in the European Pharmacopoeia, 3^{rd} edition, supplement 2000, 488. In general, Carbomers are high molecular weight polymers of acrylic acid cross-linked with polyalkenyl ethers of sugars or polyalcohols. Different types of polyacrylic acid derivatives are also described in the USP 24 / NF 19, e. g. Carbomer 910, Carbomer 934, Carbomer 934P etc. The mean differences between the commercial types are based e. g. on the type and extent of cross-linking and molecular weight.

Polyacrylic acid, cross-linked with divinyl glycol, is separately described in the USP 24 in the monograph "Polycarbophil" (USP 24, 1348) and the corresponding Calcium salts in the monograph "Calcium Polycarbophil" (USP 24, 291) respectively.

Preferred according to the invention is a polyacrylic acid derivative selected in the group of polyacrylic acid derivatives cross-linked with allyl ethers of pentaerythritol. Particularly preferred was the Carbomer 971 P.

The carbomer can be used as such or possibly after neutralising, in part or totally, its acidic carboxylic residual functions with a base, chosen in the group consisting of ammonia, triethyl amine, tromethamine, diethanolamine, triethanolamine, lysine, arginine, or possibly as a salt the cation of which is preferably selected in the group consisting of sodium, potassium, calcium, magnesium.

The excipients possibly used in the pharmaceutical compositions for nasal administration according to the invention are those commonly used in the pharmacopoeia for this purpose.

### TEST:

Three dry powder pharmaceutical compositions containing nepadutant were tested in dogs to evaluate their relative bioavailability. Four fasted Beagle dogs (2 females and 2 males), weighing 6-8 kg, were used for each composition. They were administered the formulated powders in a nostril, by blowing the dose of the container through a nasal cannula. Theoretical dose was 12 mg/kg, actual administered dose was calculated by weighing container and cannula before and after administration. Venous blood (2 ml) was collected immediately before and at 0.083, 0.25, 0.5, 0.75, 1, 2, 4, 6, 8, 24 hours after treatment, into heparinized vials. Plasma was assayed for nepadutant by means of a validated HPLC/MS-MS method, with a lower limit of quantification of 0.4 ng/ml and a linear response up to 400 ng/ml.

The results of the pharmacokinetic evaluation are reported in the following table, where also data obtained with a liquid formulation (aqueous solution; administered dose 4 mg/kg) are shown.

**Table.**

| Plasma kinetics parameters of nepadutant following intranasal administration of diverse pharmaceutical compositions to dogs. | | | | | |
|---|---|---|---|---|---|
| Reported values are normalized to the theoretical dose of 12 mg/kg. | | | | | |
| Composition Ingradients & ratio (w:w) | Physical form | Cmax (ng/ml) | Tmax (h) | AUC₀₋₂₄ (ng.h/ml) | Relative bioavailability |
| Nepadutant: carbomer, type 971P 1 : 2 | dry powder | 10656 | 1.5 | 61398 | 100% |
| Nepadutant : lactose 1 : 2 | dry powder | 294 | 0.5 | 2382 | 3.9% |
| Nepadutant : hypromellose 1 : 2 | dry powder | 488 | 1 | 2808 | 4.6% |
| Nepadutant : tween 80 : glycerol 4:2:15 % in phosphate buffer pH 6 | water solution | 679 | 0.5 | 1980 | 3.2% |

By comparison to the AUC value obtained after intravenous administration of nepadutant, the following absolute bioavailabily values could be obtained: 52, 2, 2 and 2% in the above order. The dry powder composition containing the polyacrylic acid polymer derivative Carbomer, type 971 P was definitely superior to the others. The pharmaceutical compositions which are the objects of the present inventions could be prepared according to the following non limitative examples :

| Example 1 (powder formulation): | |
|---|---|
| nepadutant | 50 % |
| Carbomer 934 P | 50 % |

| Example 2 (powder formulation) | |
|---|---|
| nepadutant | 64 % |
| Carbomer 971 P | 33 % |

| Example 3 (powder formulation) | |
|---|---|
| nepadutant | 33 % |
| Calcium Polycarbophil | 64 % |

| Example 4 (liquid formulation) | |
|---|---|
| nepadutant | 4 % |
| Tween 80 | 2 % |
| Carbomer 971 P | 3 % |
| Tromethamine | q. s. ad pH 6.5 |
| purified water | ad 100 % |

| Example 5 (liquid formulation) | |
|---|---|
| cyclo ([Asn (β--D-2-deoxy-2-amino-Glc)-Asp-Trp-Phe-Dap-Leu] cyclo (2β--5β-)) (EP0815126 example No. 3) | 2 % |
| Tween 20 | 1 % |
| Carbomer 980 | 2 % |
| NaOH | q. s. ad pH 6.5 |
| Xylitol | 3 % |
| purified water | ad 100 % |

| Example 6 (powder formulation) | |
|---|---|
| nepadutant | 35 % |
| Carbomer 971 P | 27 % |
| Tromethamine | 38 % |

| Example 7 (powder formulation) | |
|---|---|
| cyclo ( [Asn [(4-O-(α-D-Glc)-□-D-Glc)]-Asp-Trp-Phe-Dap- Leu] cyclo(2β--5β-)) (EP0815126 example No. 17) | 10 % |
| Carbomer 941 | 10 % |
| Tromethamine | 16 % |
| Lactose monohydrate | ad 100 % |

The formulations are produced with help of typical pharmaceutical procedures like milling, mixing or solving, packaging. the resulting formulations for nasal application are e. g. powders, liquids, gels or ointments. From the physicochemical point of view the resulting formulations can also include different disperse systems like e. g. suspensions or emulsions.. For application usual devices are used.

## Claims

1. A pharmaceutical composition suitable for the nasal administration containing:
i) an NK-2 antagonist or its pharmaceutically acceptable salts
ii) a polyacrylic acid and/or its derivatives
possibly in combination with other excipients used in the preparation of pharmaceutical composition for nasal administration.

2. A pharmaceutical composition according the claim 1, in which the NK-2 antagonist is selected among byciclic compounds of general formula (I) 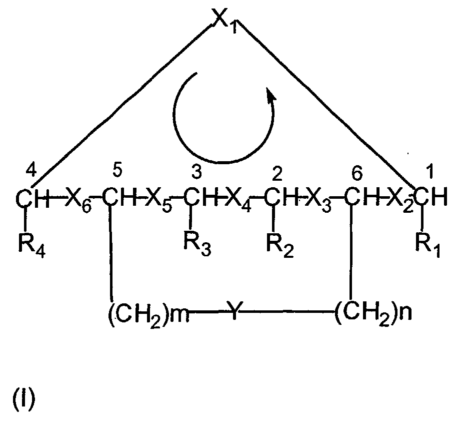 wherein:
X1, X2 , X3, X4, X5, and X6, same or different from one another, represent a - NR'CO- or a -CONR'- group, wherein R' is H or C1-3 alkyl;
Y represents a group selected from -NRCO-, -CONR-, or -SS- wherein R is H or C1-3 alkyl;
at least one of the R1, R2, R3 and R4 groups, same or different from one another, is hydrophilic and the remaining groups are hydrophobic;
m and n, same or different from one another, are each an integer number from 1 to 4.

3. A pharmaceutical composition according the claim 2, in which:
- the hydrophobic groups can be separately selected from the following:
a) groups CnH2n+1 wherein n= 0,1-4
b) linear- or branched alkyl groups corresponding to CnH2n-U-W wherein n= 1-4; U= 0, COO, CONH, S and W= C1-5 alkyl-, aryl or C1-5 alkyl-aryl-group containing from 1 to 15 carbon atoms and where aryl- is selected between phenyl or naphtyl
c) (CH2)n -C6H3-A-B wherein n= 0, 1-3; A and B, placed in any of the ortho, meta or para positions, same or different from one another, represent H, halogen, OR, NHR, NR2, CH3, SR wherein R is an C1-4 alkyl-, phenyl- or C1-4 alkyl-phenyl-group,
d) (CH2)n -C6H10 R', wherein n= 0, 1-3 and R'= H, C1-3 alkyl
e) (CH2)n -heterocycle, wherein n= 0, 1-3 and for heterocycle it is meant: imidazolyl-2-yl, indolyl-3-yl, furanyl-3-yl, pyridyl-3-yl, imidazolyl-3-yl
f) a -(CH2)s- group, wherein s= 3, 4, possibly OH-substituted or condensed with an aromatic group, which cyclizes with one of the two adjacent X1-6 groups in order to produce the side chain of proline, hydroxyproline, octahydroindol-2-carboxylic acid, tetrahydroisoquinolinic acid
g) the side chain of a natural hydrophobic amino acid selected from the group consisting of: glycine, alanine, valine, leucine, isoleucine, methionine, phenylalanine, tyrosine, tryptophan, proline, histidine, aspargine, glutamine.
h) the side chain of a natural hydrophilic amino acid, selected from the group consisting of: serine, threonine, cysteine, aspartic acid, glutamic acid, t-carboxyglutamic acid, arginine, ornithine, lysine, suitably substituted in order to render it hydrophobic through the formation of an esteric or an amidic bond with a C1-4 acyl group or a C1-3 alkyl group.
i) the side chain of non-natural hydrophobic amino acids selected from the group consisting of: norleucine, norvaline, alloisoleucine, cyclohexylglycine (Chg), α-amino-n-butyric acid (Aba), cyclohexylalanine (Cha), aminophenylbutyric acid (Pba), phenylalanines mono- and disubstituted in the ortho, meta and para positions of the benzene ring with one or more of the following groups: C1-10 alkyl, C1-10 alkoxy, halogen, β-2-thienylalanine, β-3-thienylalanine, β-2- furanyl alanine, β-3-furanylalanine, β-2-piridylalanine, β-3-piridylalanine, β-4-piridylalanine, β-(1-naphtyl)alanine, β-(2-naphtyl)alanine, O-C1-5alkylated serine- threonine- tyrosine-derivatives, S-C1-5 alkyl cysteine, S-C1-5alkyl homocysteine, N-C1-5 alkyl lysine, N-C1-5 alkyl ornithine, N-C1-5 alkyl 2,3 diaminopropionic acid.
- the hydrophilic groups are chosen in the group L-Q wherein L is a chemical bond or a linear or branched C1-6 alkyl-group and Q is chosen in the group consisting of:
i) hydroxyl, amine, guanidine, carboxyl, sulfate, phosphonate, phosphate;
ii) linear, branched or cyclic C1-6 alkyl chain containing one or more hydroxyl, amine, guanidine, carboxyl, sulfate, phosphate;
iii) an aromatic group, selected between phenyl or naphtyl, mono-, di- or trisubstituted ortho-, meta-, para-position with hydroxyl, amino, guanidine, carboxyl, sulfate, phosphate;
iv) a group M, OM, CONHM, NHCOM wherein M is an hydrophilic group
wherein the group M is chosen in the group consisting of:
a) possibly substituted mono-, di-, tri-glycosidic residues, selected from the group consisting of: D or L ribose, D or L arabinose, D or L xylose, D or L lyxose, D or L allose, D or L altrose, D or L glucose, D or L mannose, D or L gulose, D or L idose, D or L galactose,D or L talose, D or L allulose, D or L fructose, D or L sorbose, D or L tagatose; 5-deoxy-D or L-arabinose, 2-deoxy-D or L-glucose, 2-deoxy-D or L-galactose, 2-deoxy-D or L-arabinose, 2-deoxy-D or L-ribose, D or L fucose, D or L ramnose; D-glucosamine, D-mannosamine, D-galactosamine, daunosamine, acosamine and N-acylate derivative s thereof with lower fat acids, i.e. containing a N-formylic, acetylic, propionilic, butyric residue; glucuronic acid, galacturonic acid; cellobiose, lactose, maltose, D-lactosamine, cellotriose, maltotriose; tris(hydroxymethyl)methyl, D or L arabitol, D or L erythrol, D or L-perseitol, D or L ribitol, D or L sorbitol, D or L xylitol; or those where the group M is selected from the residue of tartaric acid, glucaric acid, gluconic acid, bycine, quinic acid, mucic acid, glucosaminic acid.
b) linear, branched or cyclic C1-6 alkyl-chains, containing one or more groups hydroxyl, amine, guanidine, carboxyl, sulfate, phosphonate, phosphate.

4. A pharmaceutical composition according the claim 3, in which:
- the hydrophobic group is the side chain of a natural amino acid chosen in the group consisting of: glycine, alanine, valine, leucine, isoleucine, methionine, phenylalanine, tyrosine, tryptophan, proline, histidine, aspargine, glutamine or the side chain of non-natural hydrophobic amino acids selected from the group consisting of: norleucine, norvaline, alloisoleucine, cyclohexylglycine (Chg), α-amino-n-butyric acid (Aba), cyclohexylalanine (Cha), aminophenylbutyric acid (Pba), phenylalanines mono- and di- substituted in the ortho, meta and para positions of the benzene ring with one or more of the following groups: C1-10 alkyl, C1-10 alkoxy, halogen; β-2-thienylalanine, β-3-thienylalanine, β-2- furanyl alanine, β-3-furanylalanine, β-2-piridylalanine, β-3-piridylalanine, β-4-piridylalanine, β-(1-naphtyl)alanine, β-(2-naphtyl)alanine,
- and the hydrophilic group is a group L-Q wherein L is a chemical bond or a linear or branched C1-6 alkyl-group and Q is chosen in the group consisting of: M, OM, CONHM, NHCOM wherein M is chosen in the group consisting of :
i) quinyl, gluconyl, glucoryl, sorbitolyl, sulphobenzoyl, sulphophenyl, sulphopropionyl, arginyl, lysyl,
ii) possibly substituted mono-, di-, tri-glycosidic residues selected from the group consisting of: D or L ribose, D or L arabinose, D or L xylose, D or L lyxose, D or L allose, D or L altrose, D or L glucose, D or L mannose, D or L gulose, D or L idose, D or L galactose, D or L talose, D or L allulose, D or L fructose, D or L sorbose, D or L tagatose; 5-deoxy-D or L-arabinose, 2-deoxy-D or L-glucose, 2-deoxy-D or L-galactose, 2-deoxy-D or L-arabinose, 2-deoxy-D or L-ribose, D or L fucose, D or L ramnose; D-glucosamine, D-mannosamine, D-galactosamine, daunosamine, acosamine and N-acylate derivative s thereof with lower fat acids, i.e. containing a N-formylic, acetylic, propionilic, butyric residue; glucuronic acid, galacturonic acid; cellobiose, lactose, maltose, D-lactosamine, cellotriose, maltotriose.

5. A pharmaceutical composition according the claim 4, wherein if one or both R1 and R4 groups are hydrophilic, both R2 and R3 are hydrophobic and viceversa.

6. Pharmaceutical composition according to claims 1-5 wherein the NK-2 antagonist is a compound of general Formula (I) chosen in the group consisting of:
cyclo([Asnβ-D-Glc)-Asp-Trp-Phe-Dap-Leu]cyclo(2β--5β-))
cyclo ([Asn (β--D-2-deoxy-2-amino-Glc)-Asp-Trp-Phe-Dap-Leu] cyclo (2β--5β-))
cyclo ([Asn(β--D-2-deoxy-2-acetamido-Glc)-Asp-Trp-Phe-Dap-Leu]cyclo(2β--5β-))
cyclo([Asn(β--D-mannopyranosyl)-Asp-Trp-Phe-Dap-Leu] cyclo(2β--5β-))
cyclo([Asn(β--D-galactopyranosyl)-Asp-Trp-Phe-Dap-Leu] cyclo(2β--5β-))
cyclo ([Asn(1-deoxy-sorbitol-1-yl)-Asp-Trp-Phe-Dap-Leu] cyclo(2β--5β-))
cyclo ([Asn [(4-O-(α-D-Glc)-β-D-Glc)]-Asp-Trp-Phe-Dap- Leu] cyclo(2β--5β-))
cyclo ([Asn(D-2-deoxy-glucopyranos-2-yl)-Asp-Trp-Phe-Dap-Leu] cyclo(2β--5β-))
cyclo ([Asn(β--D-2-deoxy-mannopyranos-2-yl)-Asp-Trp-Phe-Dap- Leu] cyclo(2β--5β-))
cyclo ([Dap(Lysyl)-Asp-Trp-Phe-Dap-Leu] cyclo(2β--5β-))

7. Pharmaceutical composition according to claim 6 wherein the NK-2 antagonist is cyclo ([Asn(β--D-2-deoxy-2-acetamido-Glc)-Asp-Trp-Phe-Dap-Leu]cyclo(2β--5β-))

8. Pharmaceutical composition according to claim 1 - 7 wherein the polyacrylic acid derivative is a high molecular weight polymers of acrylic acid cross-linked with polyalkenyl ethers of sugars or polyalcohols.

9. Pharmaceutical composition according to claim 8 wherein the polyacrylic acid derivative is a polyacrylic acid derivatives cross-linked with allyl ethers of pentaerythritol.

10. Pharmaceutical composition according to Claim 9 wherein the polyacrylic acid derivative is a carbomer.

11. Pharmaceutical composition according to Claim 10 wherein the carbomer is Carbomer 971P.

12. Pharmaceutical compositions according to claims 1 - 7 wherein the acidic carboxylic residual functions of the polyacrylic acid derivatives are neutralised, in part or totally, with a base chosen in the group consisting of ammonia, triethyl amine, tromethamine, diethanolamine, triethanolamine, lysine, arginine.

13. Pharmaceutical compositions according to claims 1 - 7 wherein the acidic carboxylic residual functions of the polyacrylic acid derivatives are salified, totally or partially, and present a cation selected in the group consisting of sodium, potassium, calcium, magnesium.

14. Pharmaceutical composition according to claims 1 - 13 in the form of powders.

15. Pharmaceutical composition according to claim 14 wherein the particle size distribution is comprised between 10 and 50 µm.

16. Pharmaceutical composition according to claims 1 - 13 in the form of liquid or gel.
